# EUROPEAN PATENT SPECIFICATION

(11) **EP 4 114 921 B1**
(45) Date of publication and mention of the grant of the patent: **06.05.2026**
(21) Application number: 21717327.7
(22) Date of filing: 02.03.2021
(51) Int. Cl.: C12N 5/071

(54) **METHODS FOR PREPARING KERATINOCYTES**
VERFAHREN ZUR HERSTELLUNG VON KERATINOZYTEN
PROCÉDÉS DE PRÉPARATION DE KÉRATINOCYTES

(30) Priority: 02.03.2020 EP 20305217
(43) Date of publication of application: 11.01.2023
(73) Proprietor: Centre d'Etude des Cellules Souches (CECS), 91100 Corbeil Essonnes (FR); Université Evry Val d'Essonne, 91000 Evry-Courcouronnes (FR)
(72) Inventor: BALDESCHI, Christine, 91360 Villemoisson sur Orge (FR); DOMINGUES, Sophie, 91100 Corbeil-Essones (FR); LEMAITRE, Gilles, 91080 Evry-Courcouronnes (FR)
(74) Representative: Cabinet Grosset-Fournier & Demachy
(86) International application number: PCT/EP2021/055188
(87) International publication number: WO 2021/175858

(56) References cited:
- WO-A1-2009/156398
- WO-A1-2016/039687
- WO-A1-2016/209166
- WO-A1-2017/091547
- AASEN TROND ET AL: "Isolation and cultivation of human keratinocytes from skin or plucked hair for the generation of induced pluripotent stem cells", NATURE PROTOCOLS, NATURE PUBLISHING GROUP, GB, vol. 5, no. 2, 1 January 2010 (2010-01-01), pages 371 - 382, XP009156425, ISSN: 1750-2799, DOI: 10.1038/NPROT.2009.241
- S R CHOWDHURY ET AL: "Effect of supplementation of dermal fibroblasts conditioned medium on expansion of keratinocytes through enhancing attachment", INDIAN JOURNAL OF EXPERIMENTAL BIOLOGY, 1 May 2012 (2012-05-01), India, pages 332 - 339, XP055582001, Retrieved from the Internet <URL:https://pdfs.semanticscholar.org/8bc5/f91ea4a0f4343b565f4aa50885fb50d86280.pdf>
- ANONYMOUS: "DISSOCIATION SOLUTIONS FOR ADHERENT CELL CULTURE", 25 October 2024 (2024-10-25), XP093242462, Retrieved from the Internet <URL:https://www.bioind.com/media/wysiwyg/documents/brochures/bi-usa-trypsin-brochure-10282019.pdf>
- "Antibody-drug conjugates; IN: Methods in Molecular Biology; ISSN 1064-3745; Vol. 1045", vol. 1195, 1 January 2013, HUMANA PRESS, US, ISBN: 978-1-62703-541-5, article IGOR KOGUT ET AL: "Differentiation of Human Induced Pluripotent Stem Cells into a Keratinocyte Lineage", pages: 1 - 12, XP055239563, DOI: 10.1007/7651_2013_64
- SOPHIE DOMINGUES ET AL: "Differentiation of nonhuman primate pluripotent stem cells into functional keratinocytes", STEM CELL RESEARCH & THERAPY, vol. 8, no. 1, 1 December 2017 (2017-12-01), XP055707905, DOI: 10.1186/s13287-017-0741-9
- KARL GLEDHILL ET AL: "Melanin Transfer in Human 3D Skin Equivalents Generated Exclusively from Induced Pluripotent Stem Cells", PLOS ONE, vol. 10, no. 8, 26 August 2015 (2015-08-26), pages e0136713, XP055557022, DOI: 10.1371/journal.pone.0136713
- METALLO CHRISTIAN M ET AL: "Directed differentiation of human embryonic stem cells to epidermal progenitors", ANTIBODY-DRUG CONJUGATES; IN: METHODS IN MOLECULAR BIOLOGY; ISSN 1064-3745; VOL. 1045; [METHODS IN MOLECULAR BIOLOGY; ISSN 1064-3745; VOL. 1045], HUMANA PRESS, US, vol. 585, 1 January 2010 (2010-01-01), pages 83 - 92, XP001525208, ISBN: 978-1-62703-541-5
- GANNA BILOUSOVA ET AL: "Differentiation of Mouse Induced Pluripotent Stem Cells into a Multipotent Keratinocyte Lineage", JOURNAL OF INVESTIGATIVE DERMATOLOGY, vol. 131, no. 4, 1 April 2011 (2011-04-01), NL, pages 857 - 864, XP055431278, ISSN: 0022-202X, DOI: 10.1038/jid.2010.364
- ANETA SCIEZYNSKA ET AL: "Isolation and culture of human primary keratinocytes - a methods review", EXPERIMENTAL DERMATOLOGY, vol. 28, 1 January 2019 (2019-01-01), COPENHAGEN; DK, pages 107 - 112, XP055708825, ISSN: 0906-6705, DOI: 10.1111/exd.13860

## Description

### FIELD OF THE INVENTION

The present invention relates to *ex vivo* methods for obtaining populations of human keratinocytes derived from human pluripotent stem cells.

### BACKGROUND OF THE INVENTION

The skin consists of self-renewing layers organized into functional units of differentiating cells with their origin in a single basal stratum of proliferating keratinocytes. The dead and dying cells that comprise the stratum corneum are continually shed during desquamation and replaced by cells derived from epidermal stem cells found in the stratum germinativum. Loss of epidermal function leads to loss of thermal regulation, reduced microbial defences, risks of desiccation, inhibited wound repair, and cosmetic concerns. In the absence of sufficient autologous donor for skin grafts, coverage of wounds with cultured human keratinocytes represents a promising option for treatment.

Furthermore, *in vitro* and *in vivo* models for human skin may represent tremendous tools for studying the lineage of epidermis cells or for testing cosmetic and pharmaceutical compounds for therapeutic or toxicological effects. For example the need for *in vitro* models is strengthened by the fact that there is an incentive to provide an alternative to the use of animals for testing compounds and formulations.

In addition, a number of diseases affect the function of keratinocytes, either cell autonomously or through alteration of their ability to form the pluristratified epidermal tissue. In vitro and in vivo models for human skin may represent ways to reveal molecular mechanisms of diseases and, as a consequence, identify pharmacological or biological compounds endowed with therapeutic potentials.

Thus, there is a need for methods for obtaining populations of human keratinocytes that may then be useful for skin therapy or for obtaining in vitro and *in vivo* models for human skin. Embryonic stem cells (ESC) and somatic cells that are genetically reprogrammed in order to replicate all characteristics of embryonic stem cells (such as, for example, those called "iPS" cells, for "induced pluripotent stem" cells) are pluripotent stem cells with an extensive proliferative capacity and accordingly offer a great potential use in research and medicine. Several attempts have therefore been described in the prior art for obtaining human keratinocytes from pluripotent stem cells.

To date, several groups have reported procedures to differentiate human ES/iPS cells into epidermal keratinocytes.

For example, US2009075374 describes a method of generating p63-positive cells, comprising the step of culturing embryoid bodies (EBs) in a medium comprising a retinoïd and a bone morphogenetic protein for about two days to about nine days.

WO2016061071 describes a method for providing engraftable keratinocyte stem cells by differentiation of pluripotent stem cells comprising (a) forming aggregates of the pluripotent stem cells in a suspension culture in the presence of a defined basal medium; (b) culturing the aggregates in a suspension culture in the presence of an initiation culture medium comprising retinoic acid and BMP-4 to effect the formation of initiated aggregates; (c) culturing the initiated aggregates in a keratinocyte progenitor culture medium comprising cholera toxin and a TGFβR1 kinase inhibitor to effect the formation of a cell population comprising keratinocyte progenitors; and (d) culturing the keratinocyte progenitors in a keratinocyte stem cell maturation medium to effect the formation of a cell population comprising engraftable keratinocyte stem cells.

WO2009156398 describes a method for obtaining a population of human keratinocytes derived from human pluripotent stem cells comprising a step of co-culturing human pluripotent stem cells with a layer of feeder fibroblasts in the presence of a keratinocyte culture medium supplemented with BMP-4 and ascorbic acid. The keratinocyte culture medium is further supplemented with one or more agents selected from the group consisting of glutamine, epidermal growth factor (EGF), sodium pyruvate, adenine, insulin, hydrocortisone, choleric toxin and triodothyronin. The keratinocytes obtained from the pluripotent stem cells co-express the keratinocyte markers keratin 5 (K5) and keratin 14 (K14). However, the flow cytometry analysis of the expression of K5 and K14 in keratinocytes shows the presence of two types of keratinocytes.

It is also possible to cite the following documents disclosing protocols for the preparation of a population of human keratinocytes derived from human pluripotent stem cells or methods associated with the isolation and cultivation of human primary keratinocytes, inter alia their enzymatic separation from other cells: WO2016039687, WO2016209166, WO2017091547, Differentiation of Human Induced Pluripotent Stem Cells into a Keratinocyte Lineage, I. Kogut et al., Methods in Molecular Biology, 2013; Differentiation of nonhuman primate pluripotent stem cells into functional keratinocytes, S. Domingues et al., Stem Cell Research & Therapy, 2017; Melanin Transfer in Human 3D Skin Equivalents Generated Exclusively from Induced Pluripotent Stem Cells, K. Gledhill et al., PLOS ONE; Directed differentiation of human embryonic stem cells to epidermal progenitors, C. Metallo et al. Methods in Molecular Biology, 2010; Differentiation of Mouse Induced Pluripotent Stem Cells into a Multipotent Keratinocyte Lineage, A. Sciezynska et al., Isolation and cultivation of human keratinocytes from skin or plucked hair for the generation of induced pluripotent stem cells, A.Trond et al., Nature Protocols, 2010; Effect of supplementation of dermal fibroblasts conditioned medium on expansion of keratinocytes through enhancing attach, Chowdhury S R et al. Additional background art includes the document "DISSOCIATION SOLUTIONS FOR ADHERENT CELL CULTURE Strategic portfolio of trypsin and alternative cell dissociation products for all cell types" containing a comprehensive compilation of information concerning cell dissociation from Biological Industries (www.bioind.com).

Hence, there is a need for improved methods to generate and obtain an homogeneous population of keratinocytes. Moreover, there is a need to provide such cells with high purity.

The present description provides methods that allow the production of iPSC- or ESC-derived keratinocytes needed for clinical, research or therapeutic applications that are independent of EBs, that do not use feeder cells, and above all that provide an homogeneous and pure population of keratinocytes.

### SUMMARY OF THE INVENTION

The present invention is defined in the appended claims and relates to a method for preparing keratinocytes derived from human pluripotent stem cells comprising or consisting in the steps of:
(a) forming and culturing aggregates or clumps of said pluripotent stem cells on a cell culture surface coated with a protein matrix to support cell attachment and growth in the presence of a defined human pluripotent stem cell medium;
(b) culturing the adherent aggregates or clumps of said pluripotent stem cells on a cell culture surface coated with a protein matrix in the presence of a defined keratinocyte culture medium comprising retinoic acid and BMP-4 to generate keratinocyte progenitors;
(c) culturing the keratinocyte progenitors on a cell culture surface coated with a defined protein matrix coating in the presence of a defined keratinocyte culture medium devoid of retinoic acid and BMP-4;
(d) treating the population of cells obtained in step c) to remove the non-adherent cells and non-conform cells and obtain an homogeneous population of keratinocytes,
wherein in the step d) of the method, the cells are treated a first time with trypsin or trypsin-EDTA during 2-3 minutes at 37°C to eliminate contaminant cells like fibroblasts or aged keratinocytes, and a second time with trypsin or trypsin-EDTA during 5-10 minutes to detach the keratinocyte progenitors and/or the K5/K14 positive cells exhibiting a keratinocyte-like phenotype.

### DETAILED DESCRIPTION OF THE INVENTION

In the following description and claims use will be made, with a variety of terms, and the meaning of such terms as they should be construed in accordance with the present teaching is as follows:
As used herein, the term "epithelial marker" or "KER marker", as used herein, refers to any phenotypic feature of a cell that can be used to characterize it or discriminate it from other cell types. A marker may be a protein (including secreted, cell surface, or internal proteins; either synthesized or taken up by the cell); a nucleic acid (such as an mRNA, or enzymatically active nucleic acid molecule) or a polysaccharide. Included are determinants of any such cell components that are detectable by antibody, lectin, probe or nucleic acid amplification reaction that are specific for the marker of the cell type of interest. The markers can also be identified by a biochemical or enzyme assay or biological response that depends on the function of the gene product. Specific, non-limiting examples of methods that can be used for the detection of a cell surface marker are immunohistochemistry, fluorescence activated cell sorting (FACS), and enzymatic analysis. Associated with each marker is the gene that encodes the transcript, and the events that lead to marker expression. A marker is said to be preferentially expressed in an undifferentiated or differentiated cell population, if it is expressed at a level that is at least 50% higher (in terms of total gene product measured in an antibody or PCR assay) or 30% more frequently (in terms of positive cells in the population) than an acceptable control.

The term "population of human keratinocytes" refers to a population of cells that is able to reconstruct a human epidermis and that is characterized by the capacity to produce keratins in the process of differentiating into the dead and fully keratinized cells of the stratum corneum. Markers of basal keratinocytes include markers of basal layer with keratin 5, 14 (K5/K14) and transcription factor ΔNp63, markers of supra basal layer with keratin 1 and keratin 10 (K1/K10), involucrin and fillagrin.

In a particular embodiment, the agent that stimulates epidermal induction is selected from the group consisting of Bone Morphogenetic Proteins (such as BMP-2, BMP-4 and BMP-7), receptor-regulated Smad proteins (such as Smad 1 , Smad 5 and Smad 9) and ligands of the TGF-beta family (such as Growth and Differenciation Factor 6 GFD-6). In a preferred embodiment the agent that stimulates epidermal induction is selected from the group consisting of BMP-2, BMP-4, BMP-7, Smadi, Smad5, Smad7 and GFD-6. In a more preferred embodiment, the agent that stimulates epidermal induction is BMP-4.

As used herein, the term "organotypic culture" refers to a three-dimensional tissue culture where cultured cells are used reconstruct a tissue or organ *in vitro.*

The term "cell plating" can also extend to the term "cell passaging." Cells of the description can be passaged using cell culture techniques well known to those skilled in the art. For example, this term refers to a technique that involves the steps of (1) releasing cells from a solid support or substrate and disassociation of these cells, and (2) diluting the cells in media suitable for further cell proliferation. Cell passaging may also refer to removing a portion of liquid medium containing cultured cells and adding liquid medium to the original culture vessel to dilute the cells and allow further cell proliferation. In addition, cells may also be added to a new culture vessel that has been supplemented with medium suitable for further cell proliferation.

The term "monolayer" as used herein can refer to cells that are attached to a solid support while proliferating in suitable culture conditions. A small portion of cells proliferating in a monolayer under suitable growth conditions may be attached to cells in the monolayer but not to the solid support.

"Undifferentiated", as used herein, refers to cultured cells when a substantial proportion (at least 80%, and possibly over 90% or 95%) of the cells and their derivatives in the population display characteristic markers and morphological characteristics of undifferentiated cells, distinguishing them from differentiated cells of embryo or adult origin. Cells are recognized as proliferating in an undifferentiated state when they go through at least 1 population doubling during a cultivation period of at least 3 weeks, while retaining at least about 50%, or the same proportion of cells bearing characteristic markers or morphological characteristics of undifferentiated cells after said cultivation period.

It is intended, for the purposes of the present description, that the term pluripotent stem cell embraces any cell having the capacity for self-renewal and the potential to differentiate into one or more other cell types.

As used herein, the term "human pluripotent stem cell" (hPSC) refers to any human precursor cell that has the ability to form any adult cell.

"hPSC" as used herein, refer to precursor cells of human source that have the ability to form any adult cell. Such cells are true cell lines in that they: (i) are capable of extensive proliferation *in vitro* in an undifferentiated state; and (ii) are capable of differentiation to derivatives of all three embryonic germ layers (endoderm, mesoderm, and ectoderm) even after prolonged culture. For reference only, human embryonic stem cells (hESCs) can be derived from fertilized embryos that are less than one week old (in the cleavage or blastocyte stage) or produced by artificial means (such as by nuclear transfer) that have equivalent characteristics. Other hPSC include, without being limited thereto, multipotent adult progenitor cells (MAPs), induced pluripotent stem cells (iPSC) and amniotic fluid stem cells.

hPSCs can be obtained using well-known cell-culture methods. For example, ESC can be isolated from single blastomeres of the cleavage or morula stage non-human embryo, from cleavage stage and morula non-human embryos and non-human blastocysts. Alternatively, non-fertilized human oocyte can be parthenogenetically activated to cleave and develop to the blastocyst stage.

For reference, in the present description, ES cells are not limited to a primary cell line collected from the inner cell mass, but may also be an established ES cell line. Examples of such an established ES cell line include: a cell line furnished from a cell population obtained by allowing the already established ES cell line to grow; and an ES cell line obtained by thawing a freeze-dried cell line and then culturing it. Such an established ES cell line is available without going through a step of disintegrating a fertilized egg.

For reference only, the ES cells used in the present description may be established from a single embryonic blastomere at the cleavage stage before the blastocyst stage, without impairing the generating ability of the embryo. Such ES cells can be obtained without destroying a fertilized egg.

For reference commercially available hPSCs can be also used in accordance with the invention. For reference hPSCs can be purchased for example from the UK Stem Cell Banks or the NIH human embryonic stem cells registry. For reference commercially available embryonic stem cell lines are BG01, BG02, BG03, BG04, CY12, CY30, CY92, CY10, TE03, TE32, SA-01, VUB-01, H1, H9 and RC9.

More particularly, for reference the human embryonic stem cell line RCe013-A (RC-9) derived as a failed to fertilise oocyte/1PN (pro-nuclear) embryo that was surplus to requirement or unsuitable for clinical use due to late development can be used. For reference Human embryonic stem cell (hESC) isolation, expansion and qualification was performed in a facilities whose specification, operation and monitoring complied with GMP standards enabling; i) a fully traceable procurement procedure with informed ethical consent which includes provision for commercial use, ii) detailed medical history and blood borne virus (BBV) screening of donors, and iii) compilation of a cell line history providing details on hESC manufacturing process and quality control testing regime.

For ethical reasons, the present description does not pertain to objects that may be considered as contrary to"ordre public" or morality. Therefore, in the context of the description, the terms "human embryonic stem cells" refer to human embryonic stem cells which isolation has not involved the destruction of an embryo. In other words, the terms "human embryonic stem cells" exclude human embryonic stem cells isolated by techniques that involve the destruction of an embryo.

In the context of the description, it is to be understood that any technique that does not involve the destruction of an embryo can be used, including those that are not described herein.

For reference, the embryos used for obtaining human embryonic stem cells are preferably embryos that cannot give rise to a human being, such as embryos destined to be discarded following *in vitro* fertilization (IVF) and embryos created solely for the purpose of stem cell research.

For reference, the terms "human embryonic stem cells" (hESC) preferably refer to human embryonic stem cells isolated from discarded embryos, research embryos, or preferably isolated by techniques that do not involve the destruction of an embryo.

For reference, hES cells or human iPS cells may be selected from master cell banks that may be constituted in a therapeutic purpose. In a preferred manner, hES cells or human iPS may be selected to avoid or limit immune rejection in a large segment of the human population. Typically hES cells or human iPS cells are HLA- homozygous for genes encoding major histocompatibility antigens A, B and DR, meaning that they have a simple genetic profile in the HLA repertory. The cells could serve to create a stem cell bank as a renewable source of cells that may be suitable for preparing human skin substitutes for use in cell therapy of pathologies associated with skin damage (e.g. wound, burns, irradiation, disease-related abnormalities of epidermis...). For reference, human pluripotent stem cells may carry a mutation or a plurality of mutations that are causative for a genetic disease of the human skin.

The "induced pluripotent stem cell" in the present description is a cell induced to have pluripotency by reprogramming a somatic cell by a known method and the like. Specifically, a cell induced to have pluripotency by reprogramming differentiated somatic cells such as fibroblast, peripheral blood mononuclear cell and the like by the expression of a combination of a plurality of genes selected from the group consisting of reprogramming genes including Oct3/4, Sox2, Klf4, Myc (c-Myc, N-Myc, L-Myc), Glis1, Nanog, Sall4, lin28, Esrrb and the like can be mentioned. Examples of preferable combination of reprogramming factors include (1) Oct3/4, Sox2, Klf4, and Myc (c-Myc or L-Myc), and (2) Oct3/4, Sox2, Klf4, Lin28 and L-Myc.

Induced pluripotent stem cell was established by Yamanaka et al. in mouse cell in 2006. In 2007, Induced pluripotent stem cell was also established from human fibroblast, and has pluripotency and self-renewal competence similar to those of embryonic stem cells.

The terms "differentiation", "differentiating" or "derivatives thereof" as used herein denote a process by which an unspecialized or relatively less specialized cell becomes relatively more specialized. In the context of cell ontogeny, the adjective "differentiated" is a relative term. Hence, a "differentiated cell" is a cell that has progressed further down a certain developmental pathway than the cell it is being compared with. A relatively more specialized cell may differ from an unspecialized or relatively less specialized cell in one or more demonstrable phenotypic characteristics, such as, for example, the presence, absence or level of expression of particular cellular components or products, e.g., RNA, proteins or other substances, activity of certain biochemical pathways, morphological appearance, proliferation capacity and/or kinetics, differentiation potential and/or response to differentiation signals, etc., wherein such characteristics signify the progression of the differentiation towards the relatively more specialized cell.

In the present context, the method of the description results in the progressive differentiation of human pluripotent stem cells and differentiating cells towards keratinocytes. Thus, as used herein, the term "differentiating" of differentiating cells to keratinocytes may be considered synonymous to the term "obtaining" keratinocytes from differentiating cells.

According to the present description, the (human) pluripotent stem cells are grown without a feeder cell layer by culturing the cells on a protein matrix coating in the presence of defined stem cell medium containing stabilized FGF2.

Cultures of pluripotent stem cells are described as "undifferentiated" when a substantial proportion of stem cells and their derivatives in the population display morphological characteristics of undifferentiated cells, clearly distinguishing them from differentiated cells of embryo or adult origin. Undifferentiated ES or iPS cells are recognized by those skilled in the art, and typically appear in the two dimensions of a microscopic view in colonies of cells with high nuclear/cytoplasmic ratios and prominent nucleoli. It is understood that colonies of undifferentiated cells can have neighboring cells that are differentiated.

More particularly, the present invention is defined in the appended claims and relates to a method for preparing keratinocytes derived from human pluripotent stem cells comprising or consisting in the steps of:
(a) forming and culturing aggregates or clumps of said pluripotent stem cells on a cell culture surface coated with a protein matrix to support cell attachment and growth in the presence of a defined human pluripotent stem cell medium;
(b) culturing the adherent aggregates or clumps of said pluripotent stem cells on a cell culture surface coated with a protein matrix in the presence of a defined keratinocyte culture medium comprising retinoic acid and BMP-4 to generate keratinocyte progenitors;
(c) culturing the keratinocyte progenitors on a cell culture surface coated with a defined protein matrix coating in the presence of a defined keratinocyte culture medium devoid of retinoic acid and BMP-4;
(d) treating the population of cells obtained in step c) to remove the non-adherent cells and non-conform cells and obtain an homogeneous population of keratinocytes,
wherein in the step d) of the method, the cells are treated a first time with trypsin or trypsin-EDTA during 2-3 minutes at 37°C to eliminate contaminant cells like fibroblasts or aged keratinocytes, and a second time with trypsin or trypsin-EDTA during 5-10 minutes to detach the keratinocyte progenitors and/or the K5/K14 positive cells exhibiting a keratinocyte-like phenotype

According to the present description, the population of interest or "conform" cells correspond to cells that a small pavimentous shape.

According to the present description, non-conform cells correspond to cells that have an elongated morphology or big shape.

According to the present description, the defined protein matrix solution used in culturing and maintaining human pluripotent stem cells is chosen in the group consisting of Matrigel^{™}, L7 coating^{™}, laminin, and vitronectin. Particularly, Matrigel^{™} or L7 coating^{™} is used to provide a substrate for cell culture and maintenance of human pluripotent stem cells.

According to the present description, the human stem cell medium according to the present description is a defined and serum-free medium. The defined serum-free medium refers to media with no unprocessed or unpurified serum or bovine pituitary extract (BPE) supplementation, and accordingly can include media with purified blood-derived components or animal tissue-derived components (such as growth factors). From the aspect of preventing contamination with heterogeneous animal-derived components, serum can be derived from the same animal as that of the stem cell(s).

The human stem cell medium according to the present description may contain or may not contain any alternatives to serum. The alternatives to serum can include materials that appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thiolglycerol, or equivalents thereto. Alternatively, any commercially available materials can be used for more convenience. The commercially available materials include knockout Serum Replacement (KSR), Chemically-defined Lipid concentrated (Gibco), and Glutamax (Gibco).

The human stem cell medium of the present description can also contain fatty acids or lipids, amino acids (such as non-essential amino acids), vitamin(s), growth factors, cytokines, antioxidant substances, 2-mercaptoethanol, pyruvic acid, buffering agents, and inorganic salts. The concentration of 2-mercaptoethanol can be, for example, about 0.05 to 1.0 mM, and particularly about 0.1 to 0.5 mM, but the concentration is particularly not limited thereto as long as it is appropriate for culturing the stem cell(s).

According to the present description the human stem cell medium may be a commercial chemically defined medium. The human stem cell medium may be appropriately defined depending on the medium and stem cells used. The medium according to certain aspects of the present description can be prepared using a medium used for culturing animal (human) cells as its basal medium, such as any of TeSR, Essential 8 medium Essential 8 CTS medium, StemFlex medium BME, BGJb, CMRL 1066, Glasgow MEM, Improved MEM Zinc Option, IMDM, Medium 199, Eagle MEM, aMEM, DMEM, Ham, RPMI 1640, StemPro, and Fischer's media, as well as any combinations thereof, but the medium is not particularly limited thereto as far as it can be used for culturing animal (human) cells.

For example, the following media are suitable to support hESC self-renewal:
a) StemPro^{®} hESC SFM, containing DMEM/F-12 with GlutaMAX^{™} medium, Bovine serum albumin 1.8% (BSA), 10 ng/mL stabilized basic FGF and 2-Mercaptoethanol,
b) CTS^{™} Essential 8^{™} Medium, containing 10 ng/mL stabilized basic FGF.

According to the present description the human stem cell medium is a medium suitable to support hPSC self-renewal supplemented with stabilized FGF2.

According to the present description, the day before the induction of the keratinocyte differentiation, the cells are manually subcultured and seeded at least at 1 clump/cm².

According to the present description, the culturing in step (a) is not accomplished in the presence of a myosin light chain kinase or Rho-associated kinase (ROCK - Y-27632) inhibitor.

According to the present description, the culturing in step (b) represents a step of induction (or initiation) of the keratinocyte differentiation which is initiated with two pulses of effective amounts of BMP-4 and retinoïc acid at Day 1 and Day 3 or Day 4, in the culture medium, in particular the defined keratinocyte Serum Free medium (dKSFM).

According to an embodiment of the description, the concentration of retinoic acid in the keratinocyte culture medium may vary from 0.1 µM to 10 µM. In a particular embodiment the concentration of retinoic acid is 1µM.

According to an embodiment of the description, the concentration of BMP-4 in the keratinocyte culture medium may vary from 0.02 nM to 77 nM or 0.3 ng/ml to 1000 ng/ml. In a particular embodiment the concentration of BMP-4 is 0.273 nM.

An "effective amount" is an amount sufficient to effect beneficial or desired results. An effective amount can be administered in one or more administrations. An effective amount of the can be an amount that is sufficient to promote differentiation of pluripotent stem cells into keratinocytes or expansion of keratinocyte progenitors.

According to the present description, the culturing in step (b) or step (c) is not accomplished by contacting cells with an effective amount of any one or more of the factors, including, but not limited to ascorbic acid, cholera toxin, nicotinamide, a cyclic AMP analogue (e.g., 8-Br-cAMP), and a TGF RI Kinase Inhibitor II (TGFRKi).

According to the present description, the culturing in step (b) is accomplished for a time period of 5 to 8 days, particularly 5, 6, 7 or 8 days.

According to the present description, human pluripotent stem cells (e.g. hES cells or human iPS cells) are cultivated for a time period sufficient for allowing the initiation of the differentiation of the human pluripotent stem cells into keratinocytes in a medium in presence of retinoic acid and BMP-4.

According to the present description, human pluripotent stem cells (e.g. hES cells for reference or human iPS cells) are cultivated for a time period sufficient for allowing the complete differentiation of said cells in a population of cells that recapitulate all morphological and functional attributes of human basal keratinocytes ("human keratinocytes derived from human pluripotent stem cells"). The complete differentiation of said cells into keratinocytes is accomplished in a medium without retinoic acid and BMP-4.

According to a particular embodiment, the culturing in step (b) and in step (c) is preferably accomplished in the same defined keratinocyte Serum Free medium.

According to a particular embodiment, the time period for allowing the complete differentiation of said cells into keratinocytes is from 8 days to 25 days.

According to the present description, the culturing in step (c) is accomplished for a time period of at least 8, 9, 10, 11, 12, 13, 14, 15, 16, 17, 18, 19, 20, 21, 22, 23, 24 or 25 days.

According to the present description, the method for preparing keratinocytes comprises or consists in the steps of:
(a) forming and culturing aggregates or clumps of said pluripotent stem cells on a cell culture surface coated with a protein matrix to support cell attachment and growth in the presence of a defined human pluripotent stem cell medium;
(b) culturing the adherent aggregates or clumps of said pluripotent stem cells on a cell culture surface coated with a protein matrix in the presence of a defined keratinocyte culture medium comprising retinoic acid and BMP-4 to generate keratinocyte progenitors for a time period of 5 to 8 days;
(c) culturing the keratinocyte progenitors on a cell culture surface coated with a defined protein matrix coating in the presence of a defined keratinocyte culture medium devoid of retinoic acid and BMP-4 for a time period of 8 to 25 days;
(d) treating the population of cells obtained in step c) to remove the non-adherent cells and non-conform cells and to obtained an homogeneous population of keratinocytes.

According to the present description, step d) may comprise combinations of mechanical, enzymatic and chemical treatment- e.g. using a cell scraper or trypsin-EDTA.

According to a preferred embodiment, the step d) of the method is a two-step dissociation procedure comprising or consisting in washing and treating the cells enzymatically.

According to a preferred embodiment, the step d) of the method is a two-step dissociation procedure comprising or consisting in a first treatment with trypsin to remove the non-conform cells, and a second treatment with trypsin to detach the keratinocyte progenitors, and/or the K5/K14 positive cells exhibiting a keratinocyte-like phenotype.

Preferably, cells are treated a first time with trypsin for a period of time sufficient to eliminate contaminant cells like fibroblasts or aged keratinocytes, and a second time with trypsin to detach the keratinocyte progenitors, and/or the K5/K14 positive cells exhibiting a keratinocyte-like phenotype.

Preferably, cells are first treated with trypsin for 2-3 minutes at 37°C to eliminated contaminant cells like fibroblasts or aged keratinocytes. After removing trypsin, cells are secondary treated with trypsin during 5 to 10 minutes at 37°C. The harvested cells are then amplified on a defined matrix protein coated dishes (L7 matrix from Lonza or Collagen I from collagen Solution) with dKSFM or CnT-07.HC (CELLnTEC) medium until cell banking at the end of passage 1.

According to the present description, the first enzymatic treatment with trypsin allows to remove more than 10% of cells, preferably between 10-20% of cells that are not K5 and/or K14 positive cells exhibiting a keratinocyte-like phenotype.

In a preferred embodiment the enzymatic treatment is effected with trypsin, or trypsin-EDTA.

The enzymic and non-enzymic dissociation reagents are typically removed by centrifuging before the cells are transferred to fresh culture vessels.

It can also be noted that Accutase is not used in the present method. Indeed, the use of Accutase does not allow to selectively detach the non-conform cells from the coating substrate during the first enzymatic treatment. By using Accutase, a variable number of unwanted cells are still adherent to the coating substrate. The detachment of non-conform cells and also of keratinocytes occurs with Accutase after a longer duration of treatment than with trypsin.

According to the present description, the method for preparing keratinocytes comprising more than 95, 96, 97, 98, 99% of K5+/K14+ keratinocytes further comprises
(e) culturing the detached cells of step d) corresponding to the keratinocyte progenitors and/or the keratinocytes in the presence of a culture medium.

According to the present description, the step e) of the method allows to prepare keratinocytes obtained after said culturing step comprising more than 95, 96, 97, 98, 99% of K5+/K14+ keratinocytes.

According to the present description, step (e) is suitable to obtain a substantially pure homogenous population of human keratinocytes derived from human pluripotent stem cells.

According to a particular embodiment, the culturing in step (e) is preferably accomplished in the same keratinocyte Serum Free medium than in step (b) and in step (c).

According to a particular embodiment, the culturing in step (e) is preferably accomplished in the same keratinocyte Serum Free medium than in step (b) and in step (c).

The starting cell and the differentiated cell generally have differing requirements for culture medium and conditions. It is usual to carry out at least an initial stage of culture, after introduction of the differentiation factors, in the presence of medium and under culture conditions known to be suitable for growth of the starting cell. This is followed by a subsequent period of culture in the presence of a differentiation medium and under conditions known to be suitable for the differentiated cell. After a sufficient time for differentiation, the differentiated cells may be further cultured for expansion of the differentiated cells in an expansion medium.

According to one embodiment, the expansion phase is effected for at least 2 weeks, at least 3 weeks, at least 4 weeks, at least 5 weeks, at least 6 weeks, at least 7 weeks, at least 8 weeks, at least 9 weeks or even 10 weeks. Preferably, the expansion phase is effected for 1 week - 10 weeks, more preferably 2 weeks - 10 weeks, more preferably, 3 weeks - 10 weeks, more preferably 4 weeks - 10 weeks, or 4 weeks - 6 weeks.

According to a particular embodiment, the expansion phase is accomplished in the same keratinocyte Serum Free medium than in step (b), in step (c) and in step (e) on L7 matrix coated dishes or preferably with CnT07.HC on Collagen I coated dishes.

In a preferred embodiment, during the expansion phase, the medium is changed every 2-3 days.

According to still another embodiment, the keratinocytes and/or the differentiating cells that differentiate toward keratinocytes are passaged at least 1 time during the expansion phase, at least twice during the expansion phase, at least three times during the expansion phase, at least four times during the expansion phase, at least five times during the expansion phase, or at least six times during the expansion phase.

Harvesting of the expanded population of keratinocytes and/or the differentiating cells may be effected using methods known in the art (e.g. using an enzyme such as trypsin).

In one embodiment, the passaging includes counting the cells transferred from the first culture vessel. Following counting, the predetermined number of cells is transferred to each of the further culture vessels.

A culture vessel used for culturing the cell(s) of the present disclosure can include, but is particularly not limited to: flask, flask for tissue culture, dish, petri dish, dish for tissue culture, multi dish, micro plate, micro-well plate, multi plate, multi-well plate, micro slide, chamber slide, tube, tray, CellSTACK^{®} Chambers, as long as it is capable of culturing the cells therein. The stem cells may be cultured in a volume of at least or about 0.2, 0.5, 1, 2, 5, 10, 20, 30, 40, 50 ml, 100 ml, 150 ml, 200 ml, 250 ml, 300 ml, 350 ml, 400 ml, 450 ml, 500 ml, 550 ml, 600 ml, 800 ml, 1000 ml, 1500 ml, or any range derivable therein, depending on the needs of the culture.

The human keratinocytes derived from human pluripotent stem cells obtainable by the method as above described are able to recapitulate all morphological and functional attributes of human basal keratinocytes. Indeed the inventors demonstrated that said cells are able to reconstruct a human epidermis (*in vitro* and *in vivo*) and that are characterized by the capacity to produce keratins. More particularly said cells express markers of basal keratinocytes that include markers of basal layer with keratin 5, 14 (K5/K14), transcription factor p63 or ΔNp63, and also keratin 19 (K19), which is a marker of skin stem cells.
According to the present description, the keratinocyte express cytokeratin 5, cytokeratin 14 andcytokeratin 19.

The keratinocytes of the present description can be characterized according to a number of phenotypic criteria. The criteria include but are not limited to the detection or quantification of expressed cell markers, enzymatic activity, and the characterization of morphological features and intercellular signaling.

The keratinocytes of the present description have morphological features characteristic of keratinocyte stem cells in nature. One or more such features present in a single cell are consistent with the cell being a member of the keratinocyte stem cell lineage. Cells of this description can also be characterized according to whether they express phenotypic markers characteristic of the keratinocyte lineage. General epidermal keratinocytes are characterized as expressing cytokeratin 5, cytokeratin 14 and cytokeratin 19. Within such a population, there are colony forming keratinocyte stem cells and keratinocyte progenitors on route to terminal differentiation.
A further object of the description relates to an isolated pure homogenous population of keratinocytes derived from human pluripotent stem cells optionally obtainable by the present method, wherein the keratinocytes express Keratin 19 at 99.9%.
The substantially pure homogenous population of human keratinocytes derived from human pluripotent stem cells of the description may be also suitable for preparing human epidermis.
A further object of the description relates to a human epidermis comprising a culture of the isolated pure homogenous population of keratinocytes derived from human pluripotent stem cells optionally obtainable by the present method, wherein the keratinocytes express Keratin 19 at 99.9%.

In a particular embodiment, human epidermis substitutes according to the description may be generated as described by Poumay, Y et al. 2004. Culture of the substantially pure homogenous population of human keratinocytes derived from human pluripotent stem cells of the description may be performed on polycarbonate culture inserts. These cells may be maintained for 5 to 7 days in CnT07.HC medium (amplification phase) and 14 days in 3D Prime CnT medium (CELLnTEC) for stratification. The cells were exposed to the air-liquid interface by removing the culture medium in the insert for 14 days for stratification. In a particular embodiment, the substantially pure homogenous population of human keratinocytes derived from human pluripotent stem cells is previously seeded on a cell culture polycarbonate insert for 5 to 7 days in CnT07.HC medium. Organotypic cultures are then grown submerged up to keratinocyte confluence, and finally maintained at the air-liquid interface for 14 days to enhance stratification and differentiation of the epithelium. For *in vivo* testings, human epidermis substitutes according to the description may be generated with the substantially pure homogenous population of human keratinocytes derived from human pluripotent stem cells is previously seeded on a plasma based matrix (fibrin) for 7 to 10 days in CnT07.HC medium to obtain a monolayer of keratinocytes (KER).

A further object of the description relates to a human epidermis substitutes obtainable by the method as above described.

A further object of the description relates to a method for grafting an animal (*in vivo* testings), preferably a mammal, more preferably a mouse, with a human skin substitute as described above. In a particular embodiment said animal is an immunodeficient animal (e.g. NOD/SCID or Nude mouse). Said method may be useful to provide animal models for human skin.

In a particular embodiment, animals grafted with a human skin substitute of the description may be generated as described by Del Rio M. et al. (2002). Briefly, animals are shaved and aseptically cleansed. Full-thickness wounds are then created on the dorsum of mice and finally grafting with the human skin substitute of the description is performed under sterile conditions. 10-12 weeks may be then sufficient to obtain a human skin on said animal.

In a particular embodiment, animals grafted with a human skin substitute of the description may be generated by "in house" method. Briefly, animals are shaved and aseptically cleansed. Full-thickness wounds are then created on the dorsum of mice and finally applied with the human skin substitute of the description is performed under sterile conditions. Faty gauzes are put on the graft area. The graft and the fatty gauzes are put inside the mouse skin borders to avoid suturing. The piece of mouse skin is devitalized by manual liquid nitrogen and PBS1X bath cycles and resutured on the graft to provide the graft with a "biological" dressing. 10-12 weeks may be then sufficient to obtain a human skin on said animal.

A further object of the description relates to an non-human animal model for human skin obtainable according to the method as above described.

The human skin substitutes and animal models of the present description may have a variety of uses. These uses include, but are not limited to, use for screening compounds, substrates for culturing tumors and pathological agents (e.g., human papilloma virus), and for modelling human injuries or pathologies associated with skin damage.

For example human skin substitutes and animal models of the present description may be used for a variety of *in vitro* and *in vivo* tests. In particular but in non limiting way, the human skin substitutes and animal models of the present description find use in the evaluation of: skin care products, drug metabolism, cellular responses to test compounds, wound healing, phototoxicity, dermal irritation, dermal inflammation, skin corrosivity, and cell damage. Typically, for animal models of the description, the product may be administered topically on the human skin, or may be administered through an oral, sublingual, subcutaneous, intramuscular, intravenous, and transdermal route.

The present descriptionencompasses a variety of screening assays. In some embodiments, the screening method comprises providing a human skin substitute or an animal model of the present description and at least one test compound or product (e.g., a skin care product such as a moisturizer, cosmetic, dye, or fragrance; the products can be in any from, including, but not limited to, creams, lotions, liquids and sprays), applying the product or test compound to said human skin substitute or animal model , and assaying the effect of the product or test compound on the human skin substitute or animal model. Typically, for animal models of the description, the test compound or product may be administered topically on the human skin, or may be administered through an oral, sublingual, subcutaneous, intramuscular, intravenous, and transdermal route. A wide variety of assays may be used to determine the effect of the product or test compound on the human skin substitute or animal model. The assays may be directed to the toxicity, potency, or efficacy of the compound or product. Additionally, the effect of the compound or product on growth, barrier function, or tissue strength can be tested.

In other preferred embodiments, the human skin substitutes or animal models of the description find use for screening the efficacy of drug introduction across the skin. In a particular embodiment, the human skin substitutes or animal models of the present description are also useful for the culture and study of tumours that occur naturally in the skin as well as for the culture and study of pathogens that affect the skin. Accordingly, in some embodiments, it is contemplated that the human skin substitutes or animal models of the present description are seeded with malignant cells. These reconstructed human skin substitutes or animal models can then be used to screen compounds or other treatment strategies (e.g., radiation or tomotherapy) for efficacy against the tumour in its natural environment. In some embodiments of the present description provide methods comprising providing a reconstructed human skin substitute or animal model infected with a pathogen of interest and at least one test compound or treatment and treating the skin substitute or animal model with the test compound or treatment.

In another particular embodiment, the human skin substitutes or animal models of the present description are also useful for modelling human injuries or pathologies associated with skin damage. For example, the human skin substitutes and animal models of the present description may provide both in vitro and in vivo models for modelling wounds, bums (e.g. fire burns, sunburns...), or lesions caused by irradiations, pathogens..., irritations caused by chemical products or environment conditions, degenerative diseases and genetic diseases. In certain embodiments, pathologies of interest are genodermatosis such as Epidermolysis bullosa, Xeroderma pigmentosum, ichthyosis, ectodermal dysplasia, kindler syndrome, Sickle cell leg ulcer and others.

Typically, the human skin substitutes or animal models of the present description may be generated from pluripotent stem cells that may carry a mutation or a plurality of mutations that are causative for a genetic disease of the human skin. Both *in vitro* and *in vivo* models as described above may have particular interests for medical research or may be useful for screening compounds for the treatment or the prevention of said injuries and pathologies.

In particular, the present description contemplates the use of the human skin substitutes and animal models according to the description for screening of compounds from libraries, in particular combinatorial libraries, using e.g. high throughput or high content techniques. Typically, for animal models of the description, the test compound or product may be administered topically on the human skin, or may be administered through an oral, sublingual, subcutaneous, intramuscular, intravenous, and transdermal route.

In a further aspect of the description, the human skin substitutes of the present description may be used for the treatment of a pathology associated with skin damage. Therefore the present description relates to a method for the treatment of a pathology associated skin damage comprising a step consisting of grafting a patient in need thereof with a human skin substitute of the description. For example, the human skin substitutes of the present description find use in wound closure and burn treatment applications. The use of grafts for the treatment of burns and wound closure is described, for example in U.S. Pat. Nos. 5,693,332. Accordingly, the present description provides methods for wound closure, including wounds caused by burns, comprising providing a human skin substitute according to the present description and a patient suffering from a wound and grafting the patient with the human skin substitute under conditions such that the wound is closed.

The substantially pure homogenous population of human keratinocytes derived from human pluripotent stem cells obtained according to the method of the description is then suitable for skin therapy.

The description relates to a pharmaceutical composition comprising a substantially pure homogenous population of human keratinocytes derived from human pluripotent stem cells of the description and optionally a pharmaceutically acceptable carrier or excipient. In certain embodiments, a pharmaceutical composition may further comprise at least one biologically active substance or bioactive factor.

As used herein the term "biologically active substance or bioactive factor" refers to any molecule or compound whose presence in a pharmaceutical composition of the description is beneficial to the subject receiving the composition. As will be acknowledged by one skilled in the art, biologically active substances or bioactive factors suitable for use in the practice of the present description may be found in a wide variety of families of bioactive molecules and compounds. For example, a biologically active substance or bioactive factor useful in the context of the present description may be selected from anti-inflammatory agents, anti-apoptotic agents, immunosuppressive or immunomodulatory agents, antioxidants, growth factors, and drugs.

A related aspect of the description relates to a method for treating a subject suffering from a pathology associated with skin damage, said method comprising a step of administering to the subject an efficient amount of a substantially pure homogenous population of human keratinocytes derived from human pluripotent stem cells of the description (or a pharmaceutical composition thereof).

As used herein, the term "efficient amount" refers to any amount of a substantially pure homogenous population of human keratinocytes derived from human pluripotent stem cells (or a pharmaceutical composition thereof) that is sufficient to achieve the intended purpose.

The substantially pure homogenous population of human keratinocytes derived from human pluripotent stem cells (or a pharmaceutical composition thereof) of the description may be administered to a subject using any suitable method. The substantially pure homogenous population of human keratinocytes derived from human pluripotent stem cells of the description may be implanted alone or in combination with other cells, and/or in combination with other biologically active factors or reagents, and/or drugs. As will be appreciated by those skilled in the art, these other cells, biologically active factors, reagents, and drugs may be administered simultaneously or sequentially with the cells of the description.

In certain embodiments, a treatment according to the present description further comprises pharmacologically immunosuppressing the subject prior to initiating the cell-based treatment. Methods for the systemic or local immunosuppression of a subject are well known in the art. Effective dosages and administration regimens can be readily determined by good medical practice based on the nature of the pathology of the subject, and will depend on a number of factors including, but not limited to, the extent of the symptoms of the pathology and extent of damage or degeneration of the tissue or organ of interest, and characteristics of the subject (e.g., age, body weight, gender, general health, and the like).

The invention will be further illustrated by the following figures and examples.

### BRIEF DESCRIPTION OF THE DRAWINGS

**Figure 1** :
   (A) Schematic representation of the first protocol design for differentiation of hESCs into keratinocytes according to a method adapted from the prior art WO2009156398 (here, the cells are cultivated on a coating matrix , whereas in WO2009156398, the cells are cultivated on feeder cells). Keratinocyte differentiation was induced with BMP-4 (0,5 nmol/L) and L-ascorbic acid (AA) (0,03 mmol/L) throughout the kinetic. At Day 0, hESCs are manually passaged on clinical grade matrix in clinical grade GREEN medium supplement with BMP-4 and AA. Cells are maintained in culture until D40. At D40, the keratinocytes are isolated using trypsin treatment and amplified until banking at passage 1 (p1).
   (B) Schematic representation of the clinical and feeder free protocol design for differentiation of hESCs RC-9 into keratinocytes. At Day 0, the RC-9 are mechanically seeded into new dishes coated with L7 matrix and cultured in hESC condition with StemPro hESC medium supplemented with 10ng/mL of stabilized FGF-2. The induction of KER differentiation is initiated with two pulses of BMP-4 (0,273nmol/L) and retinoic acid (1µmol/L) at Day 1 and Day 5, until Day 6, in defined Keratinocyte Serum Free medium (dKSFM). At Day 7 until the end of the induction of differentiation process, cells are cultured in differentiation medium composed with dKSFM medium only. At the end of differentiation, cells are isolated and purified with differential trypsinizination.
**Figure 2** **:**
   (A) Representative fluorescence-activated cell sorting analysis of keratin 5 and keratin 14 in keratinocytes KER-RC9 and NHKs at passage 2 (p2) (B1-A and B2-A correspond to a relative fluorescence average in a specific channel depending secondary antibody fluorochromes). Those data illustrate that K5 present different expression profiles between KER-RC9 and NHKs. In NHKs, the population is homogenous with only one K5 and K14 peak whereas in KER-RC9, cells are homogenous for K14 marker but two distinct populations are present for K5 marker, suggesting the presence of 2 two types of KER-RC9 in the population (according to a method adapted from the prior art WO2009156398)
   (B) Representative fluorescence-activated cell sorting analysis of keratin 5 and keratin 14 in keratinocytes KER-RC9 at p2 (B1-A and B2-A correspond to a relative fluorescence average in a specific channel depending secondary antibody fluorochromes). The FACS analysis show that the population is homogenous with only one K5 and K14 peak. KER-RC9 present a pure population expressing the two keratinocyte markers keratin 5 and keratin 14 at more than 98% (respectively 100% and 100%).
   (C) Representative fluorescence-activated cell sorting analysis of K8/18, ΔNp63, K5 and K14 in cells during the differentiation (B1-A, B2-A and R1-A correspond to a relative fluorescence average in a specific channel depending on conjugated antibodies fluorochromes APC, FITC and PE/555). During the process, cells were analysed in order to determine the end of differentiation step. Cells highly express K8/K18 markers (epithelial markers, not expressed by adult keratinocytes but present in embryo skin) at the beginning of the differentiation. The K8/K18 signal is progressively lost and at D20 the majority of cells do not express those markers. Cells express ΔNp63 after 10 days of differentiation and it is stable until the end. It is followed up with keratin 5 and keratin 14. Those two markers appeared progressively during the differentiation and are stable after 15 days of differentiation. The increase in keratin 5 and 14 is concomitant with the decrease of keratin 8/18.
   (D) Representative fluorescence-activated cell sorting analysis of Keratin19, (R1-A correspond to a relative fluorescence average in a specific channel depending on conjugated antibody fluorochrome APC). The FACS analysis show that the population is homogenous for KER-RC-9 (K-hESC) with the presence of a pure population expressing Keratin 19 at 99.9% whereas the primary KER (HPEK), only a small fraction of cells express Keratin 19 (28.2%) suggesting stemness of KER-RC9.
**Figure 3** :
   (A) Haematoxylin-eosin staining and immuno-histological analysis of *in vitro* epidermal reconstitutions of KER-RC9 at p2 on polycarbonate insert or on plasma based matrix containing human primary fibroblasts as equivalent dermis. In the two *in vitro* epidermis culture system, KER-RC9 are capable to reconstruct a pluristratified epidermis with a basal layer, a spinous layer, a granular layer and finally a corneal layer. Those reconstructed stratified epidermis express basal keratinocyte marker as keratin 5 and the suprabasal marker keratin 10.
   (B) Haematoxylin-eosin staining and immunohistological analysis of in vivo of KER-RC9 at p2 seeded on plasma based matrix (without primary fibroblasts) grafted on the back of a nude mouse during 12 weeks. After 10-12 weeks, human epidermis is detected with a specific suprabasal human Involucrin marker. This antibody fail to detect mouse involucrin whereas the keratin 10 antibody detect all species (human and mouse). The epidermis is pluristratified with all four layer of human epidermis like in in vitro assay. To conclude, KER-RC9 are fully functional *in vitro* as well *as in vivo.*

### EXAMPLES:

### Manual hESCs culture (reference example)

Clinical-grade hESC line RCe013-A (RC-9), was used and cultured in feeder free conditions using StemPro^{™} hESC Medium (Invitrogen) and stabilized FGF2 (Miltenyi) at 10ng/ml and L7^{™} coating (Lonza). Cells were plated and grown until they reached 80 percent of confluence.

### Differentiation of hES cells in keratinocytes (reference example)

Clumps of hESC were seeded in the defined Keratinocyte SFM (dKSFM- Gibco - Thermo Fisher Scientific) which is a serum-free medium optimized for the isolation and expansion of human keratinocytes without the need for bovine pituitary extract (BPE) supplementation or the use of fibroblast feeder layers. The induction of ectodermal differentiation was done when 0.273 nM of human recombinant BMP-4 (Peprotech) and 1µM of trans retinoic acid (Sigma) were added. Cells were grown in the same medium until clones of epithelial cells were isolated.

During all the differentiation process the medium was changed every 2/3 days.

At the end of differentiation, cells are isolated and purified with differential trypsinizination. Briefly, cells are first treated with trypsin for 2-3 minutes in 37°C to eliminated contaminant cells like fibroblasts. After discard trypsin, cells are secondary treated with new trypsin during 5 to 10 minutes at 37°C. The harvested cells are then amplified on collagen I coated dishes with CnT-07.HC medium or on L7 matrix dishes with dKSFM medium until cell banking at the end of passage 1.

Microscopy analysis of hESC RC9 line during keratinocyte differentiation process. The RC-9 colonies growth and differentiate until D6. At Day 10, the differentiating colonies are still growing and present 2 distinct morphologies : the centre part is composed of small cells and the outer part (like a ring around the centre part), is composed of migrating bigger cells. After D15, the outer part is bigger : cells escape from the centre. After D20, the majority of cells are out of the centre of colonies and the centre of colonies is composed with cells bigger than at D6.

Representative fluorescence-activated cell sorting analysis of K8/18, ΔNp63, K5, K14 and optionally K19 in cells during the differentiation (B1-A, B2-A and R1-A correspond to a relative fluorescence average in a specific channel depending on conjugated antibodies fluorochromes APC, FITC and PE/555). During the process, cells were analysed in order to determine the end of differentiation step. Cells highly express K8/K18 markers (epithelial markers, not expressed by adult keratinocytes) at the beginning of the differentiation. The K8/K18 signal is progressively lost and at D20 the majority of cells do not express those markers. Cells express ΔNp63 after 10 days of differentiation and it is stable until the end. It is followed up with keratin 5, keratin 14 and optionally keratin 19. Those two markers appeared progressively during the differentiation and are stable after 15 days of differentiation. The increase in keratin 5 and 14 is concomitant with the decrease of keratin 8/18.

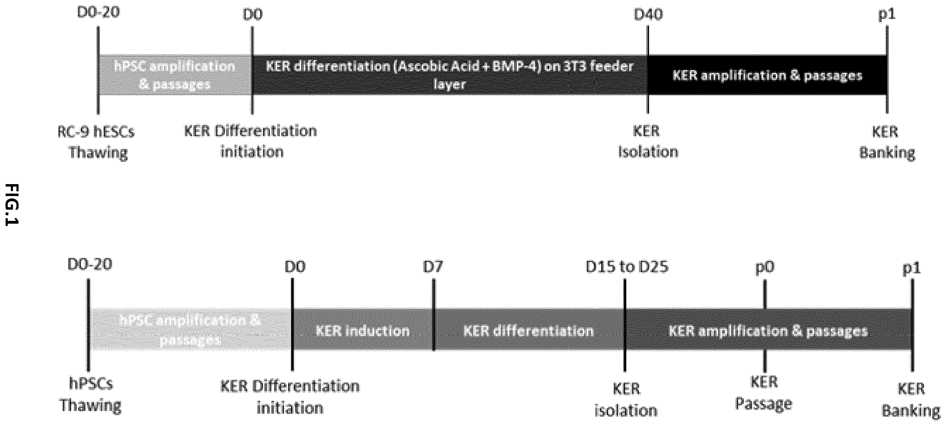

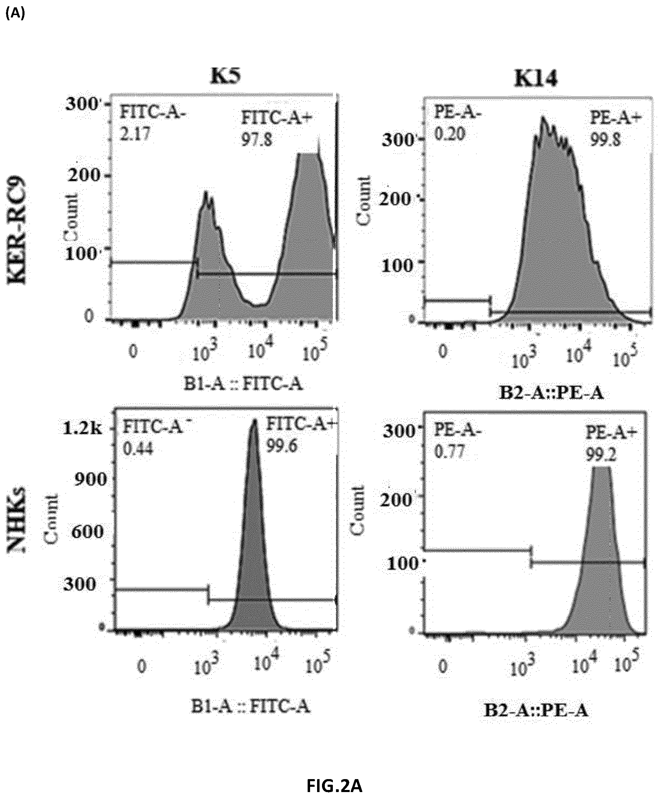

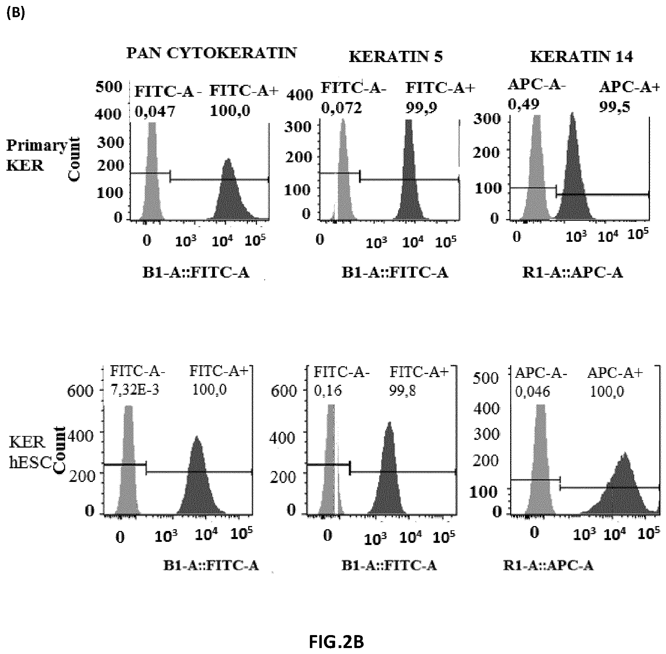

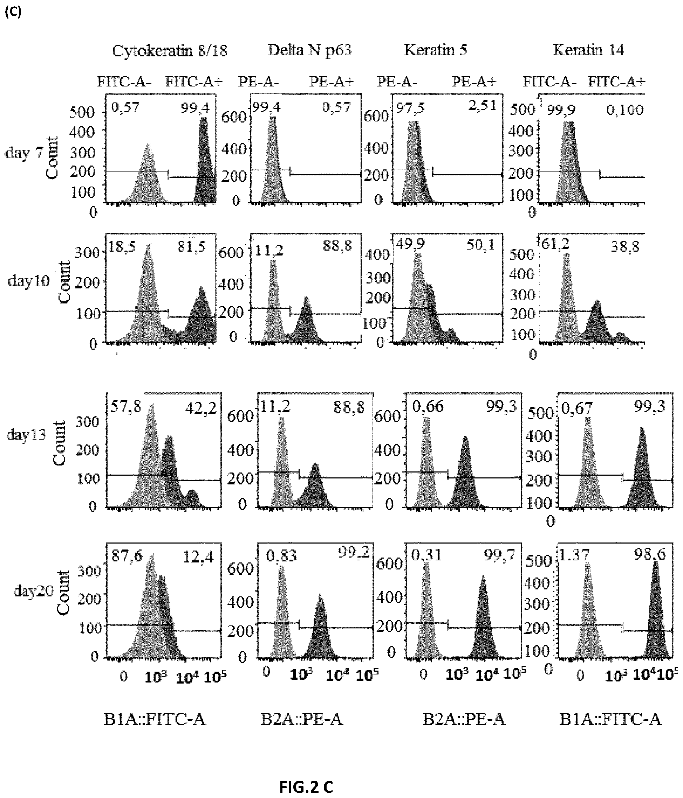

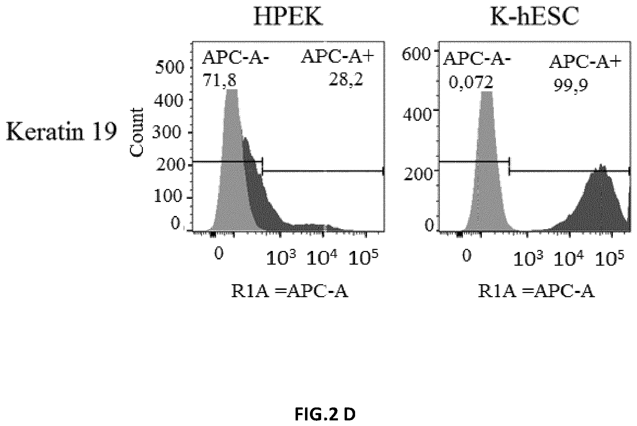

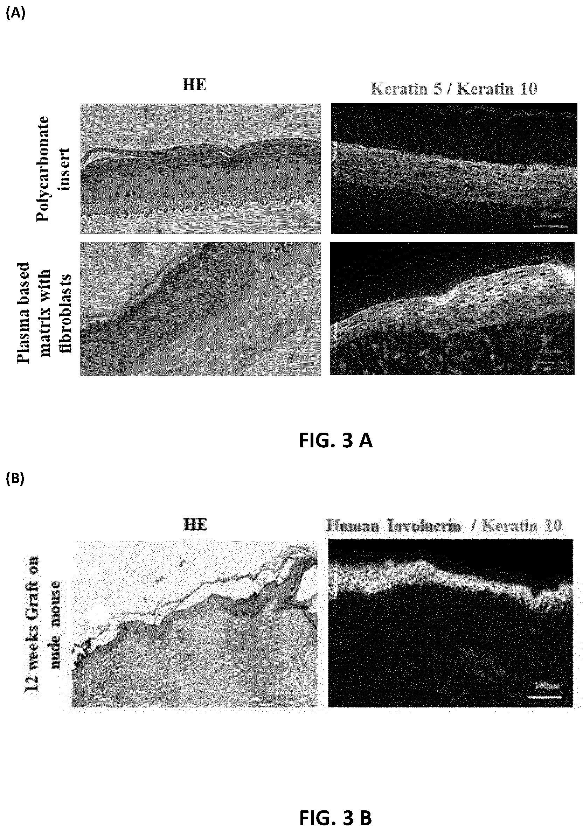

As shown in Figure 2C, the methods according to the present description allow the preparation of iPSC- or ES-derived keratinocytes that provide an homogeneous and pure population of keratinocytes.

### FACS analyses

Cells were detached from culture plates using Trypsin-EDTA (Invitrogen) and fixed and permeabilized using BD CytoFix/CytoPerm kit (BD Biosciences) according to the manufacturer instruction. Conjugated antibodies diluted at 1 : 50 were incubated 15 to 30 min at room temperature in PBS containing 0.1 % FCS. Control samples were done using specific isotype.Stained cells were analyzed on a MACS Quant flow cytometer (Miltenyi) using FlowJo software.

### Immunocytochemistry

Cells were fixed in 4% paraformaldehyde for 15 minutes at room temperature before permeabilized and blocking in PBS supplement with 0.4% Triton X-100 and 5% BSA (Sigma). Primary antibodies were incubated overnight at 4°C in blocking buffer. Rabbit anti-K14 is from Covance and, mouse anti-K5 is from Abcam. Cells were stained with the species specific fluorophore-conjugated secondary antibody (Invitrogen) for 1 hour at room temperature and nucleus were dye using DAPI. Immunofluorescence images were acquired on a Zeiss Z2 microscope using Axiovision imaging software.

### Organotypic cultures

Human epidermis substitutes are reconstructed according a modified version of described protocol by Poumay, Y et al. 2004. Culture of the keratinocytes derived from human pluripotent stem cells is performed on polycarbonate culture inserts. These cells may be maintained for 5 to 7 days in CnT07.HC medium (amplification phase) and 14 days in 3D Prime CnT medium (CELLnTEC) for stratification. The cells were exposed to the air-liquid interface by removing the culture medium in the insert for 14 days for stratification.

For *in vivo* testings, human epidermis substitutesare generated with keratinocytes derived from human pluripotent stem cells previously seeded on a plasma based matrix (fibrin) for 7 to 10 days in CnT07.HC medium to obtain a monolayer of keratinocytes (KER). For grafting, nude mice are aseptically cleansed. Full-thickness wounds are then created on the dorsum of mice and finally applied with the human skin substitute of the description is performed under sterile conditions. Fatty gauzes are put on the graft area. The graft and the fatty gauzes are put inside the mouse skin borders to avoid suturing. The piece of mouse skin is devitalized by liquid nitrogen and PBS1X bath cycles and resutured on the graft to provide the graft with a "biological" dressing. 10-12 weeks may be then sufficient to obtain a human skin on said animal. Implants were harvested between 4-12 weeks after grafting, and the tissue specimens fixed in 10% buffered formalin for paraffin embedding.

## Claims

1. A method for preparing keratinocytes derived from human pluripotent stem cells comprising or consisting in the steps of:
(a) forming and culturing aggregates or clumps of said pluripotent stem cells on a cell culture surface coated with a protein matrix to support cell attachment and growth in the presence of a defined human pluripotent stem cell medium;
(b) culturing the adherent aggregates or clumps of said pluripotent stem cells on a cell culture surface coated with a protein matrix in the presence of a defined keratinocyte culture medium comprising retinoic acid and BMP-4 to generate keratinocyte progenitors;
(c) culturing the keratinocyte progenitors on a cell culture surface coated with a defined protein matrix coating in the presence of a defined keratinocyte culture medium devoid of retinoic acid and BMP-4;
(d) treating the population of cells obtained in step c) to remove the non-adherent cells and non-conform cells and obtain an homogeneous population of keratinocytes,
wherein in the step d) of the method, the cells are treated a first time with trypsin or trypsin-EDTA during 2-3 minutes at 37°C to eliminate contaminant cells like fibroblasts or aged keratinocytes, and a second time with trypsin or trypsin-EDTA during 5-10 minutes to detach the keratinocyte progenitors and/or the K5/K14 positive cells exhibiting a keratinocyte-like phenotype.

2. The method for preparing keratinocytes according to claim 1, wherein the human stem cell medium is a medium suitable to support hESC self-renewal supplemented with stabilized FGF2.

3. The method for preparing keratinocytes according to claim 1 or 2, wherein the culturing in step (b) comprises two pulses of effective amounts of BMP-4 and retinoic acid introduced in the culture medium at Day 1 and Day 3 or Day 4.

4. The method for preparing keratinocytes according to any one of claims 1 to 3, wherein the culturing in step (b) is accomplished for a time period of 5 to 8 days.

5. The method for preparing keratinocytes according to any one of claims 1 to 4, wherein the culturing in step (c) is accomplished for a time period of at least 8, 9, 10, 11, 12, 13, 14, 15, 16, 17, 18, 19, 20, 21, 22, 23, 24 or 25 days.

6. The method for preparing keratinocytes according to any one of claims 1 to 5, comprising or consisting in the steps of:
(a) forming and culturing aggregates or clumps of said pluripotent stem cells on a cell culture surface coated with a protein matrix to support cell attachment and growth in the presence of a defined human pluripotent stem cell medium;
(b) culturing the adherent aggregates or clumps of said pluripotent stem cells on a cell culture surface coated with a protein matrix in the presence of a defined keratinocyte culture medium comprising retinoic acid and BMP-4 to form keratinocyte progenitors for a time period of 5 to 8 days;
(c) culturing the keratinocyte progenitors on a cell culture surface coated with a defined protein matrix coating in the presence of a defined keratinocyte culture medium devoid of retinoic acid and BMP-4 for a time period of 8 to 25 days;
(d) treating the population of cells obtained in step c) to remove the non-adherent cells and non-conform cells and to obtain an homogeneous population of keratinocytes.

7. The method for preparing keratinocytes according to any one of claims 1 to 6, wherein the method for preparing keratinocytes further comprises
(e) culturing the detached cells of step d) corresponding to the keratinocyte progenitors and/or the keratinocytes in the presence of a culture medium, and wherein the keratinocytes obtained after said culturing step comprise more than 95, 96, 97, 98, 99% of K5+/K14+ keratinocytes.

8. The method for preparing keratinocytes according to any one of claims 1 to 7, wherein the keratinocyte express cytokeratin 5, cytokeratin 14 and cytokeratin 19.

## Patentansprüche

1. Verfahren für die Herstellung von Keratinozyten, die von menschlichen pluripotenten Stammzellen abgeleitet sind, umfassend die Schritte oder bestehend aus den Schritten des:
(a) Bildens und Züchtens von Ansammlungen oder Klumpen der pluripotenten Stammzellen auf einer Zellkulturfläche, die mit einer Proteinmatrix beschichtet ist, um Zellanhaftung und Wachstum in Gegenwart eines definierten menschlichen pluripotenten Stammzellenmediums zu unterstützen;
(b) Züchtens der anhaftenden Ansammlungen oder Klumpen der pluripotenten Stammzellen auf einer Zellkulturfläche, die mit einer Proteinmatrix beschichtet ist, in Gegenwart eines definierten Keratinozytkulturmediums, das Retinolsäure und BMP-4 umfasst, um Keratinozyterzeuger zu erzeugen;
(c) Züchtens der Keratinozyterzeuger auf einer Zellkulturfläche, die mit einer definierten Proteinmatrixbeschichtung beschichtet ist, in Gegenwart eines definierten Keratinozytkulturmediums, das frei von Retinolsäure und BMP-4 ist;
(d) Behandelns der Population von Zellen, die in Schritt c) erhalten worden ist, um die nichtanhaftenden Zellen und nichtkonformen Zellen zu entfernen und eine homogene Population von Keratinozyten zu erhalten,
wobei in Schritt d) des Verfahrens die Zellen ein erstes Mal mit Trypsin oder Trypsin-EDTA während 2-3 Minuten bei 37 °C behandelt werden, um verunreinigende Zellen, wie Fibroblaste oder gealterte Keratinozyten, zu eliminieren und ein zweites Mal mit Trypsin oder Trypsin-EDTA während 5-10 Minuten, um die Keratinozyterzeuger und/oder die positiven K5/K14-Zellen abzulösen, die einen keratinozytähnlichen Phänotyp aufweisen.

2. Verfahren für die Herstellung von Keratinozyten nach Anspruch 1, wobei das menschliche Stammzellenmedium ein Medium ist, das geeignet ist, die hESC-Selbsterneuerung, die mit FGF2 ergänzt ist, zu unterstützen.

3. Verfahren für die Herstellung von Keratinozyten nach Anspruch 1 oder 2, wobei das Züchten in Schritt (b) zwei Pulse wirksamer Mengen von BMP-4 und Retinolsäure, die in das Kulturmedium an Tag 1 und Tag 3 oder Tag 4 eingeführt werden, umfasst.

4. Verfahren für die Herstellung von Keratinozyten nach einem der Ansprüche 1 bis 3, wobei das Züchten in Schritt (b) über eine Zeitspanne von 5 bis 8 Tagen ausgeführt wird.

5. Verfahren für die Herstellung von Keratinozyten nach einem der Ansprüche 1 bis 4, wobei das Züchten in Schritt (c) während eines Zeitraums von mindestens 8, 9, 10, 11, 12, 13, 14, 15, 16, 17, 18, 19, 20, 21, 22, 23, 24 oder 25 Tagen ausgeführt wird.

6. Verfahren für die Herstellung von Keratinozyten nach einem der Ansprüche 1 bis 5, umfassend die Schritte oder bestehend aus den Schritten des:
(a) Bildens und Züchtens von Ansammlungen oder Klumpen der pluripotenten Stammzellen auf einer Zellkulturfläche, die mit einer Proteinmatrix beschichtet ist, um Zellanhaftung und Wachstum in Gegenwart eines definierten menschlichen pluripotenten Stammzellenmediums zu unterstützen;
(b) Züchtens der anhaftenden Ansammlungen oder Klumpen der pluripotenten Stammzellen auf einer Zellkulturfläche, die mit einer Proteinmatrix beschichtet ist, in Gegenwart eines definierten Keratinozytkulturmediums, das Retinolsäure und BMP-4 umfasst, um Keratinozyterzeuger während einer Zeitspanne von 5 bis 8 Tagen zu bilden;
(c) Züchtens der Keratinozyterzeuger auf einer Zellkulturfläche, die mit einer definierten Proteinmatrixbeschichtung beschichtet ist, in Gegenwart eines definierten Keratinozytkulturmediums, das frei von Retinolsäure und BMP-4 ist, während einer Zeitspanne von 8 bis 25 Tagen;
(d) Behandelns der Population von Zellen, die in Schritt c) erhalten worden ist, um die nichtanhaftenden Zellen und nichtkonformen Zellen zu entfernen und eine homogene Population von Keratinozyten zu erhalten.

7. Verfahren für die Herstellung von Keratinozyten nach einem der Ansprüche 1 bis 6, wobei das Verfahren für die Herstellung von Keratinozyten ferner umfasst
(e) Züchten der losgelösten Zellen von Schritt d), die den Keratinozyterzeugern und/oder den Keratinozyten entsprechen, in Gegenwart eines Kulturmediums und wobei die Keratinozyten, die nach dem Züchtungsschritt erhalten worden sind, mehr als 95, 96, 97, 98, 99 % K5+/K14+-Keratinozyten umfassen.

8. Verfahren für die Herstellung von Keratinozyten nach einem der Ansprüche 1 bis 7, wobei der Keratinozyt Cytokeratin 5, Cytokeratin 14 und Cytokeratin 19 exprimiert.

## Revendications

1. Une méthode de préparation des kératinocytes dérivés de cellules souches pluripotentes humaines comprenant ou consistant en les étapes :
(a) former et cultiver des agrégats ou amas desdites cellules souches pluripotentes sur une surface de culture cellulaire recouverte d'une matrice protéique pour soutenir l'attachement et la croissance cellulaire en présence d'un milieu défini pour cellules souches pluripotentes humaines;
(b) cultiver les agrégats ou amas adhérents desdites cellules souches pluripotentes sur une surface de culture cellulaire recouverte d'une matrice protéique en présence d'un milieu de culture défini pour kératinocytes comprenant de l'acide rétinoïque et du BMP-4 pour générer des progéniteurs kératinocytaires ;
(c) cultiver les progéniteurs kératinocytaires sur une surface de culture cellulaire recouverte d'un revêtement de matrice protéique défini en présence d'un milieu de culture défini pour kératinocytes, dépourvu d'acide rétinoïque et de BMP-4 ;
(d) traiter la population de cellules obtenue à l'étape c) pour éliminer les cellules non adhérentes et les cellules non conformes et obtenir une population homogène de kératinocytes,
dans laquelle, à l'étape d) de la méthode, les cellules sont traitées une première fois avec de la trypsine ou de la trypsine-EDTA pendant 2 à 3 minutes à 37°C pour éliminer les cellules contaminantes telles que les fibroblastes ou les kératinocytes âgés, puis une seconde fois avec de la trypsine ou de la trypsine-EDTA pendant 5 à 10 minutes pour détacher les progéniteurs kératinocytaires et/ou les cellules K5/K14 positives présentant un phénotype de type kératinocyte.

2. La méthode de préparation des kératinocytes selon la revendication 1, dans laquelle le milieu cellulaire humain est un milieu adapté à l'auto-renouvellement des hESC complété par du FGF2 stabilisé.

3. La méthode de préparation des kératinocytes selon la revendication 1 ou 2, dans laquelle l'étape cultiver (b) comprend deux pulses de quantités efficaces de BMP-4 et d'acide rétinoïque introduits dans le milieu de culture au jour 1 et au jour 3 ou jour 4.

4. La méthode de préparation des kératinocytes selon l'une des revendications 1 à 3, dans laquelle l'étape cultiver (b) est effectuée pendant une période de 5 à 8 jours.

5. La méthode de préparation des kératinocytes selon l'une des revendications 1 à 4, dans laquelle l'étape cultiver (c) est effectuée pendant une période d'au moins 8, 9, 10, 11, 12, 13, 14, 15, 16, 17, 18, 19, 20, 21, 22, 23, 24 ou 25 jours.

6. La méthode de préparation des kératinocytes selon l'une des revendications 1 à 5, comprenant ou consistant en les étapes :
(a) former et cultiver des agrégats ou amas desdites cellules souches pluripotentes sur une surface de culture cellulaire recouverte d'une matrice protéique pour soutenir l'attachement et la croissance cellulaire en présence d'un milieu défini pour cellules souches pluripotentes humaines;
(b) cultiver les agrégats ou amas adhérents desdites cellules souches pluripotentes sur une surface de culture cellulaire recouverte d'une matrice protéique en présence d'un milieu de culture défini pour kératinocytes comprenant de l'acide rétinoïque et du BMP-4 pour générer des progéniteurs kératinocytaires pendant une période de 5 à 8 jours ;
(c) cultiver les progéniteurs de kératinocytes sur une surface de culture cellulaire recouverte d'un revêtement de matrice protéique défini en présence d'un milieu de culture défini pour kératinocytes, dépourvu d'acide rétinoïque et de BMP-4 , pendant une période de 8 à 25 jours ;
(d) traiter la population de cellules obtenue à l'étape c) pour éliminer les cellules non adhérentes et non conformes et obtenir une population homogène de kératinocytes.

7. La méthode de préparation des kératinocytes selon l'une des revendications 1 à 6, dans laquelle la méthode de préparation des kératinocytes comprend en outre
(e) cultiver les cellules détachées de l'étape d) correspondant aux progéniteurs kératinocytaires et/ou aux kératinocytes en présence d'un milieu de culture, et dans laquelle les kératinocytes obtenus après cette étape de culture comprennent plus de 95, 96, 97, 98, 98, 99 % des kératinocytes K5+/K14+.

8. La méthode de préparation des kératinocytes selon l'une des revendications 1 à 7, dans laquelle les kératinocytes expriment la cytokératine 5, la cytokératine 14 et la cytokératine 19.
